# EUROPEAN PATENT SPECIFICATION

(11) **EP 4 193 150 B1**
(45) Date of publication and mention of the grant of the patent: **13.11.2024**
(21) Application number: 21763132.4
(22) Date of filing: 05.08.2021
(51) Int. Cl.: G01N 33/569

(54) **DETECTION DEVICE, METHOD, KIT, APPARATUS AND SYSTEM FOR DETECTING PRESENCE OF PATHOGENIC MICROORGANISMS AND CORRESPONDING ANTIBODIES IN A BIOLOGICAL SAMPLE**
NACHWEISVORRICHTUNG, VERFAHREN, KIT, VORRICHTUNG UND SYSTEM ZUM NACHWEIS PATHOGENER MIKROORGANISMEN UND ENTSPRECHENDER ANTIKÖRPER IN EINER BIOLOGISCHEN PROBE
DISPOSITIF DE DÉTECTION, PROCÉDÉ, KIT, APPAREIL ET SYSTÈME POUR DÉTECTER LA PRÉSENCE DE MICRO-ORGANISMES PATHOGÈNES ET D'ANTICORPS CORRESPONDANTS DANS UN ÉCHANTILLON BIOLOGIQUE

(30) Priority: 06.08.2020 IT 202000019531
(43) Date of publication of application: 14.06.2023
(73) Proprietor: Eltek S.p.A., 15033 Casale Monferrato (AL) (IT)
(72) Inventor: MELIOLI, Giovanni, 15033 Casale Monferrato (Alessandria) (IT); PIZZI, Marco, 15033 Casale Monferrato (Alessandria) (IT); GALLO, Valentina, 15033 Casale Monferrato (Alessandria) (IT); ZANIN, Massimo, 15033 Casale Monferrato (Alessandria) (IT)
(74) Representative: Buzzi, Notaro & Antonielli d'Oulx S.p.A.
(86) International application number: PCT/IB2021/057209
(87) International publication number: WO 2022/029682

(56) References cited:
- LIN QIUYUAN ET AL: "Microfluidic Immunoassays for Sensitive and Simultaneous Detection of IgG/IgM/Antigen of SARS-CoV-2 within 15 min", vol. 92, no. 14, 2 July 2020 (2020-07-02), pages 9454 - 9458, XP055788211, ISSN: 0003-2700, Retrieved from the Internet <URL:https://pubs.acs.org/doi/pdf/10.1021/acs.analchem.0c01635> DOI: 10.1021/acs.analchem.0c01635
- LIN QIUYUAN ET AL: "Supporting information Microfluidic Immunoassays for Sensitive and Simultaneous Detection of IgG/IgM/Antigen of SARS-CoV-2 within 15 min", 21 July 2020 (2020-07-21), XP055788855, Retrieved from the Internet <URL:https://pubs.acs.org/doi/suppl/10.1021/acs.analchem.0c01635/suppl_file/ac0c01635_si_001.pdf> [retrieved on 20210323]
- YANG K S ET AL: "Microfluidic chip with porous anodic alumina integrated with PDMS/glass substrate for immuno-diagnosis", CURRENT APPLIED PHYSICS, ELSEVIER, AMSTERDAM, NL, vol. 9, no. 2, 1 March 2009 (2009-03-01), pages e60 - e65, XP026196902, ISSN: 1567-1739, [retrieved on 20090313], DOI: 10.1016/J.CAP.2008.12.031
- PIRES NUNO MIGUEL MATOS ET AL: "On-site, parallel detection of bio-analytes in water by an integrated capillary flow based opto-microfluidic device", 2015 IEEE INTERNATIONAL SYMPOSIUM ON MEDICAL MEASUREMENTS AND APPLICATIONS (MEMEA) PROCEEDINGS, IEEE, 7 May 2015 (2015-05-07), pages 100 - 105, XP033168284, DOI: 10.1109/MEMEA.2015.7145180

## Description

### Field of the invention

The present invention generally relates to the field of the detection of the presence of pathogenic microorganisms and/or of corresponding antibodies in biological samples. The invention was developed with particular reference to the detection of SARS-COV-2 virus and the corresponding immunoglobulin A.

### State of the prior art

Devices and methods for detecting the presence of viruses or proteins in biological samples, collected from human or animal subjects, are known in the prior art.

In particular, devices and methods based on the immobilisation of possible viruses or proteins present in biological samples analysed on physical media, such as for example strips, which comprise binding agents specific thereto, are known. In this regard, see for example documents RU 2707526 C1, TW 202009486 A, CN 110672845 A and CN 109187968 A. Although sensitive and specific, the solutions described in these documents have the drawback of not being capable of determining the level of infectivity of the individual from whom the biological sample analysed was collected, should the analysis be intended to determine the presence of a virus or antibody in the biological sample.

Interest in devices of the type described above has been further strengthened recently, due to the onset of the COVID-19 pandemic.

This pandemic is caused by the SARS-CoV-2 virus, a virus belonging to the Coronavirus family, that is a family of viruses known to cause the onset of diseases of the respiratory tract ranging from common cold to Severe Acute Respiratory Syndrome (SARS). In particular, Coronaviruses are spherical viruses and comprise, among the various constituent elements thereof, S-Glycoprotein (Spike Glycoprotein), which determines the virus specificity for epithelial cells of the respiratory tract.

Currently known techniques for identifying infection from COVID-19 in an individual provide for the detection of the presence of the SARS-CoV-2 virus, for example by means of a Reverse Transcriptase - Polymerase Chain Reaction (RT-PCR) analysis, or the detection of the antibody response that develops in the host following contact with the virus, for example by means of immunoenzymatic assays.

RT-PCR analyses comprise the steps of reverse transcribing the RNA molecules of the virus into corresponding DNA molecules, which are then amplified. Though these analyses are extremely sensitive, they have the drawback of being slow and necessarily have to be carried out in a laboratory. Furthermore, the RT-PCR analyses allow to identify infected individuals, but the level of virulence and infectivity of such individuals cannot be determined.

On the other hand, immunoenzymatic assays are rapid tests, and there are versions that can be used even outside laboratories, without the need for supervision by specialised personnel. Such assays are designed to detect the presence of immunoglobulins of type A (IgA), M (IgM) or G (IgG) which are produced by the individual in response to viral infection. However, they have the drawback of being poorly sensitive and poorly specific. Furthermore, they detect the presence of possible immunoglobulins produced by the individual in defence against the virus, but they do not detect the presence of the virus itself. As a result, the level of virulence and infectivity of individuals who are positive to the test cannot be determined in this case either.

There is therefore a need for solutions free from the disadvantages described above.

Qiuyuan Lin et al. "Microfluidic Immunoassays for Sensitive and Simultaneous Detection of IgG/IgM/Antigen of SARS-CoV-2 within 15 min", Analytical Chemistry, Vol. 92, no. 14, 2 July 2020, pages 9454-9458, discloses a portable microfluidic immunoassay system for detection of IgG/IgM/Antigen of SARS-CoV-2 in a biological sample, wherein a detection device has three separate portions each provided with a corresponding sample port and outlet port, and wherein each separate portion is used for detecting individually IgC, IgM or the pathogens antigen.

Yang K S et al. "Microfluidic chip with porous anodic alumina integrated with PDMS/glass substrate for immune-diagnosis", Current Applied Physics, Elsevier, Amsterdam NL, vol. 9, no. 2, 1 march 2009,pages e60-e65, discloses a system for diagnosis of disease marker molecules in microfluidic devices employing anodic aluminum oxide.PIRES NUNO MIGUEL MATOS et al. "On-site, parallel detection of bi-analytes in water by an integrated capillary flow based opto-microfluidic device ", 2015 IEEE International Symposium on Medical Measurement and Application (MEMEA) Proceedings, IEEE, 7 may 2015, pages 100-105, discloses an integrated capillary-flow driven opto-microfluidic device for on-site, parallel detection of bio-analytes.

### Aim and summary of the invention

In the general terms, the present invention aims to solve one or more of the aforementioned drawbacks, by means of devices and systems for the detection of the presence of at least one pathogenic microorganism and/or of at least one corresponding antibody in a biological sample.

In this general context, a first aim of the present invention is to provide a such detection device that is easy and efficient in use.

According to a second aim, the present invention aims to provide one such detection device that is simple and cost-effective to produce.

According to a third aim, the present invention aims to provide one such detection device which is provided with high sensitivity and specificity for the at least one pathogenic microorganism and/or for the at least one corresponding antibody whose presence is to be detected, preferably provided with high sensitivity and specificity for both.

According to a fourth aim, the present invention aims to provide one such detection device capable of allowing to rapidly obtain the detection results.

According to a fifth aim, the present invention aims to provide one such device capable of allowing to distinguish the infected individuals who are contagious from the infected individuals whose infectivity is null or low.

According to a sixth aim, the present invention aims to provide a kit for detecting the presence of at least one pathogenic microorganism and/or of at least one corresponding antibody in a biological sample that is practical to use and which can also be used outside hospital facilities, without the need for supervision by specialised personnel.

According to the present invention, one or more of the aforementioned aims are achieved by a device, a kit and a system for detecting presence of at least one pathogenic microorganism and/or of at least one corresponding antibody, having the features of claims 1, 11 and 15, respectively.

The claims are an integral part of the technical teaching provided herein in relation to the invention.

### Brief description of the drawings

Further aims, characteristics and advantages of the invention will be apparent from the detailed description that follows, carried out with reference to the attached drawings, provided purely by way of non-limiting example, wherein:
- figure 1 is a schematic perspective view of a detection device according to possible embodiments of the invention;
- figure 2 is a partially sectioned schematic perspective view of a detection device according to possible embodiments of the invention;
- figure 3 is an exploded schematic view of a detection device according to possible embodiments of the invention;
- figure 4 is a sectional, partial and schematic view of a detection device according to possible embodiments of the invention;
- figure 5 is a schematic perspective view of a kit comprising a detection device according to possible embodiments of the invention;
- figure 6 is a schematic perspective view of components of a kit according to particularly advantageous embodiments of the invention;
- figure 7 is a partially sectioned schematic perspective view of a kit comprising a detection device according to possible embodiments of the invention;
- figure 8 is a detail of figure 7 in enlarged scale;
- figures 9 and 10 are partial and sectional schematic representations aimed at illustrating two operative positions which can be taken by a deviator device part of a detection device according to possible embodiments of the invention,
- figure 11 is a schematic perspective view of a detection system comprising a device or a detection kit according to possible embodiments of the invention, and a relative analysis apparatus falling outside the scope of the invention;
- figure 12 is a partially sectioned schematic perspective view of an analysis apparatus which can be used in a system of the type shown in figure 11, with a detection kit according to possible embodiments of the invention associated thereto, also partially sectioned;
- figure 13 is a schematic perspective view of an analysis apparatus falling outside the scope of the invention, which can be used in a system of the type shown in figure 11, with a detection kit according to possible embodiments of the invention associated thereto;
- figure 14 is a partially sectioned schematic perspective view of a part of an analysis apparatus according to figure 13; and
- figure 15 is a schematic perspective view of a binding or detection member which can be used in a detection device according to possible embodiments of the invention.

### Description of the preferred embodiments

Reference to an embodiment in this description indicates that a particular configuration, structure, or characteristic described regarding the embodiment is comprised in at least one embodiment. Therefore, phrases like "in an embodiment", "in various embodiments" and the like, possibly present in various parts of this description, do not necessarily refer to the same embodiment. Furthermore, particular shapes, structures or characteristics defined in this description may be combined in any suitable manner in one or more embodiments, even different from those shown. The numerical and spatial references (such as "upper", "lower", "high", "low", etcetera) used herein are for convenience only and they do not therefore define the scope of protection or scope of the embodiments.

Unless otherwise specified, the expressions "pathogenic microorganism" or "pathogen" as used in the present description relate to any biological agent responsible for the onset of a disease condition in a host organism, including viruses, for example viruses belonging to the Coronavirus family, viruses that cause the onset of diseases of the respiratory tract, spherical viruses and/or viruses comprising S-Glycoprotein (Spike glycoprotein) such as the SARS-CoV-2 virus.

Unless otherwise specified, the expression "antibody" as used in the present description relates to a protein produced by a subject in response to an infection by a pathogenic microorganism, for example an infection caused by a virus. Examples of antibodies are immunoglobulins, for example immunoglobulins of type A (IgA), M (IgM) or G (IgG).

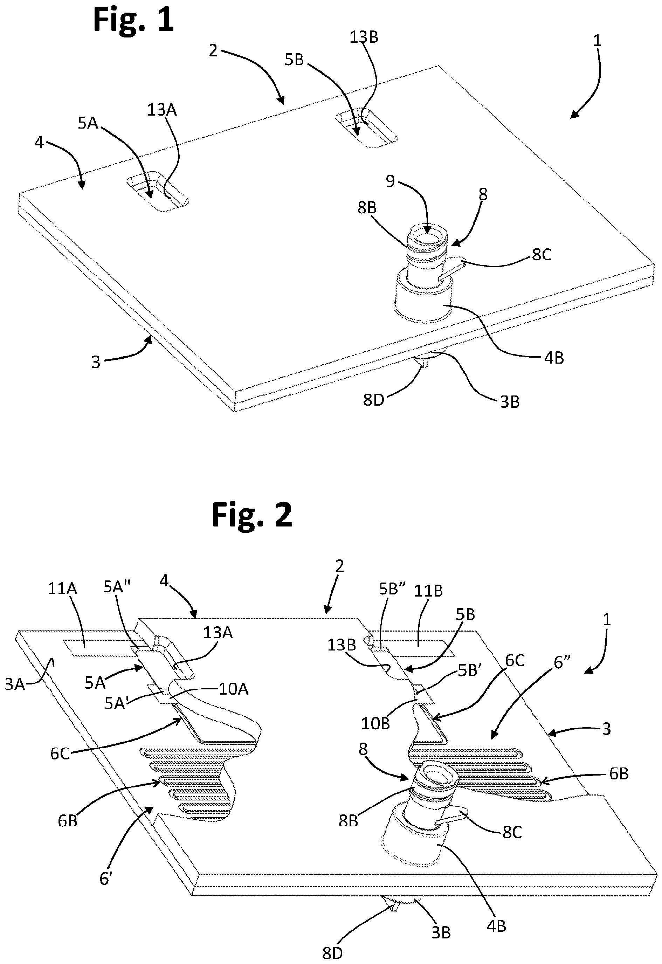

Initially with reference to figures 1 and 2, a device for detecting the presence of at least one pathogenic microorganism and/or of at least one corresponding antibody in a biological sample is indicated as a whole with reference numeral 1.

In various embodiments, the device 1 may be configured to detect presence of multiple pathogenic microorganisms and/or multiple corresponding antibodies simultaneously. However, hereinafter in the present description, reference will be made to a preferred embodiment, in which the device 1 is used to detect at least one of a single pathogenic microorganism and a single corresponding antibody. As explained in greater detail below, when using the device 1, situations may actually occur in which a biological sample collected from a subject can lead to the detection of both the pathogen and the corresponding antibody, or of the pathogen alone, or even of the antibody alone: for example, in the first case, the subject can be considered infected and not contagious, in the second case the subject can be considered infected and contagious, and in the third case the subject can be considered no longer infected (or healed) and not contagious.

Preferably, the pathogenic microorganism whose presence is detected in the biological sample analysed by means of the device 1 is a virus, more preferably a virus belonging to the Coronavirus family. In preferred embodiments, the pathogenic microorganism whose presence is detected in the biological sample analysed is the SARS-CoV-2 virus, that is the virus responsible for the COVID-19 pandemic (or a possible mutation of such virus).

Preferably, the antibody corresponding to the pathogenic microorganism whose presence is detected in the biological sample analysed by means of the device 1 is an immunoglobulin selected from among immunoglobulin A (IgA), immunoglobulin G (IgG) and immunoglobulin M (IgM). In preferred embodiments, the antibody corresponding to the pathogenic microorganism whose presence is detected in the biological sample analysed by means of the device 1 is an immunoglobulin A (IgA).

Preferably, the biological sample analysed by means of the device 1 is a fluid or substantially fluid sample selected from among saliva, fluid obtained from nasal swab and fluid obtained from pharyngeal swab. In various preferred embodiments, the biological sample analysed by means of the device 1 is saliva.

Referring in particular to the embodiments represented in figures 1 and 2, the device 1 comprises a body 2, preferably in the form of a rectangular-based parallelepiped. It is to be understood that the body 2 may also have a shape different from the one represented, for example in cylindrical-shaped, or parallelepiped-shaped with a different base, for example square-shaped.

In various embodiments, the body 2 is formed by at least two parts coupled together, preferably sealingly. In the illustrated non-limiting example, the body 2 comprises a first structure 3, which will be referred to hereinafter also as "lower structure", and a second structure 4, which will be referred to hereinafter also as "upper structure". The lower structure 3 and the upper structure 4 are preferably substantially plate-like and sealingly coupled together. The structures 3 and 4 are preferably formed by plastic material and obtained by means of moulding, for example injection moulding. Other materials and other manufacturing techniques are however not ruled out. For example, materials which can be used are polycarbonate, polystyrene, COC or other polymers known to be suitable for injection moulding which are preferably transparent.

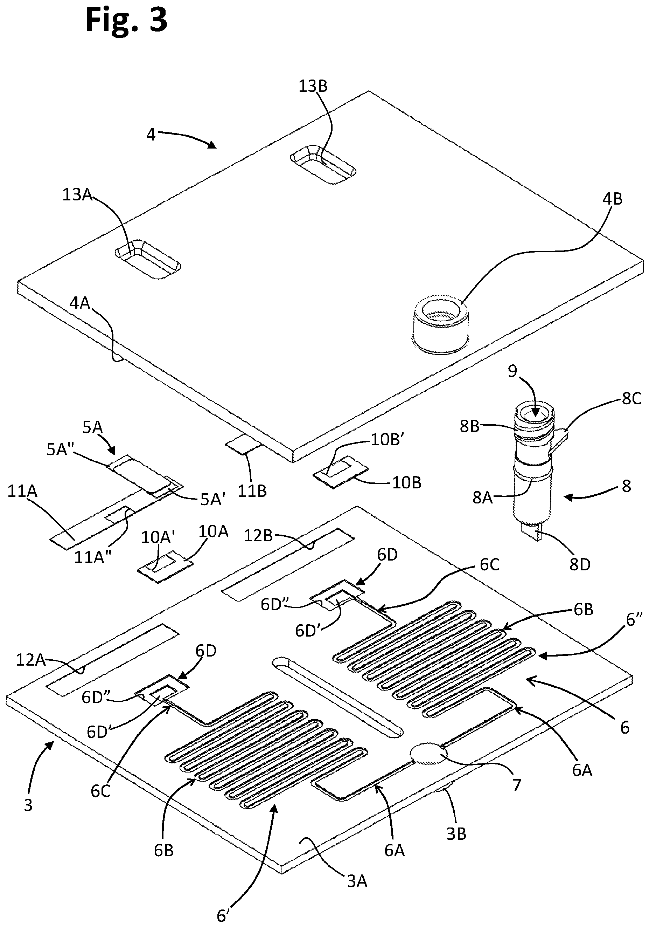

With reference also to figure 3, in the exemplified case, the lower structure 3 and the upper structure 4 are coupled together so that a surface 3A of the lower structure 3, which will be referred to hereinafter as the "upper surface" of the lower structure 3, is at contact with a surface 4A of the upper structure 4, which will be referred to hereinafter as the "lower surface" of the upper structure 4.

According to the invention, the device includes at least two binding members, prearranged to bind the pathogenic microorganism and/or the corresponding antibody whose presence is to be detected in the biological sample analysed. Preferably, the at least two binding members are equal to each other. Given that the purpose of the binding members is to allow the detection of the pathogenic microorganism and/or the corresponding antibody and/or the complexes thereof in the biological sample, hereinafter they will also be identified as "detection members".

Referring for example to figure 2, a first binding or detection member 5A and a second binding or detection member 5B are arranged in an intermediate position with respect to the two structures 3 and 4, particularly mounted on the upper surface 3A of the structure 3, for example as explained hereinafter. In various embodiments, the position of such detection members 5A and 5B is defined by respective positioning elements, which are preferably also suitable to allow and/or promote the advancement of respective fractions of the biological fluid sample toward the detection members, as explained hereinafter.

It is to be understood that the device 1 can be provided with even more than two detection members 5A, 5B. However, hereinafter in the present description reference will be made to a preferred embodiment, in which only two detection members 5A, 5B are provided for.

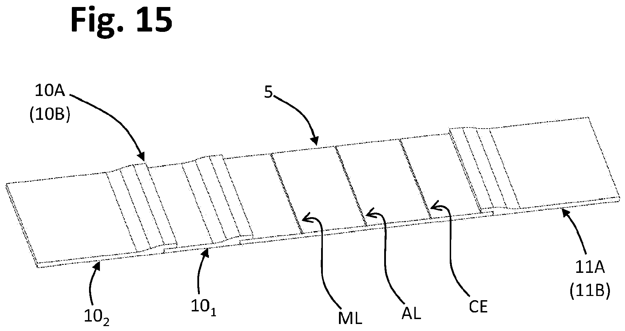

In preferred embodiments, the first detection member 5A and the second detection member 5B each comprise a corresponding strip, particularly made of substantially porous material, which includes - thereon - at least one binding element of the pathogenic microorganism to be detected, at least one binding element of the corresponding antibody and optionally at least one control element, designed to control the correct operation of the analysis. The binding elements of the pathogenic microorganism and of the antibody and the optional control element are not visible in the figures, given that they are microscopic elements not visible to the naked eye (the same are however schematised in figure 15, described hereinafter).

It is to be understood that each strip or detection member may comprise more than one binding element of the pathogenic microorganism to be detected and/or more than one binding element of the corresponding antibody and/or more than one control element. However, hereinafter in the present description, reference will be made to a preferred embodiment, wherein each strip or detection member comprises a binding element of the pathogenic microorganism to be detected, a binding element of the corresponding antibody and a control element.

In embodiments in which the pathogenic microorganism to be detected in the biological sample analysed is a virus, the binding element of the pathogenic microorganism comprises for example at least one substance selected from among a monoclonal antibody, a polyclonal antiserum and a purified or recombinant natural extractive ligand, and the binding element of the antibody comprises, for example, at least one substance selected from among a polyclonal antiserum, a monoclonal antibody and a natural ligand.

In the preferred embodiments, in which the pathogenic microorganism whose presence is sought in the biological sample analysed is the SARS-CoV-2 virus and the corresponding antibody is an IgA, the binding element of SARS-CoV-2 virus comprises, for example, at least one substance selected from among a monoclonal antibody, a polyclonal antiserum and a natural or recombinant ligand, for example ACE2, and the IgA binding element comprises at least one substance selected from among a monoclonal antibody, an anti-IgA polyclonal antiserum or an IgA natural ligand, such as the natural or recombinant spike glycoprotein.

The at least two detection members are connected to respective sections or branches of a fluidic circuit of the device 1, along which respective fractions of the biological sample are advanced. In various embodiments, the fluidic circuit comprises a surface structuring of at least one part of the body of the device, or the body 2 in the example considered herein. In various embodiments, the aforementioned structuring includes at least one shaped surface recess, defined at one of two facing surfaces of two different parts of the body of the device. For example, in particular with reference to figure 3, a fluidic circuit, indicated as a whole with 6, designed to advance the biological sample therein, is defined at the upper surface 3A of the lower structure 3.

According to the invention, the fluidic circuit 6 comprises at least one introduction portion, indicated with 7 in figure 3 alone, for the biological sample to be analysed, from which at least two circuit sections or branches extend.

Referring for example to figure 3, in various embodiments, the fluidic circuit 6 comprises at least one first branch 6' and a second branch 6", which extend substantially parallel from the introduction portion 7, in order to supply respective fractions of the biological sample to the first detection member 5A and to the second detection member 5B, respectively. The first branch 6' is therefore designed to advance a first fraction of the biological sample from the introduction portion 7 to the first detection member 5A, while the second branch 6" is designed to advance a second fraction of the biological sample from the introduction portion 7 to the second detection member 5B. In various embodiments, the two circuit branches 6' and 6" are substantially equal to each other.

In various embodiments in which there is provided for a single introduction portion from which several circuit branches extend, a deviator device, preferably comprising a deviator member of the angularly rotatable or rotating type, is operative at the introduction portion. To this end, in various embodiments the body of the device defines a housing for a such deviator member.

Referring for example to figure 1-3, each structure 3 and 4 defines a respective part 3B, 4B of a housing for a deviator member indicated with 8 as a whole, preferably also performing functions of input for the biological sample. In the example, the parts 3B, 4B are substantially cylindrical-shaped and each define a through cylindrical cavity, with the cavity of one of such two parts (part 3B in this case) which forms the aforementioned introduction portion 7.

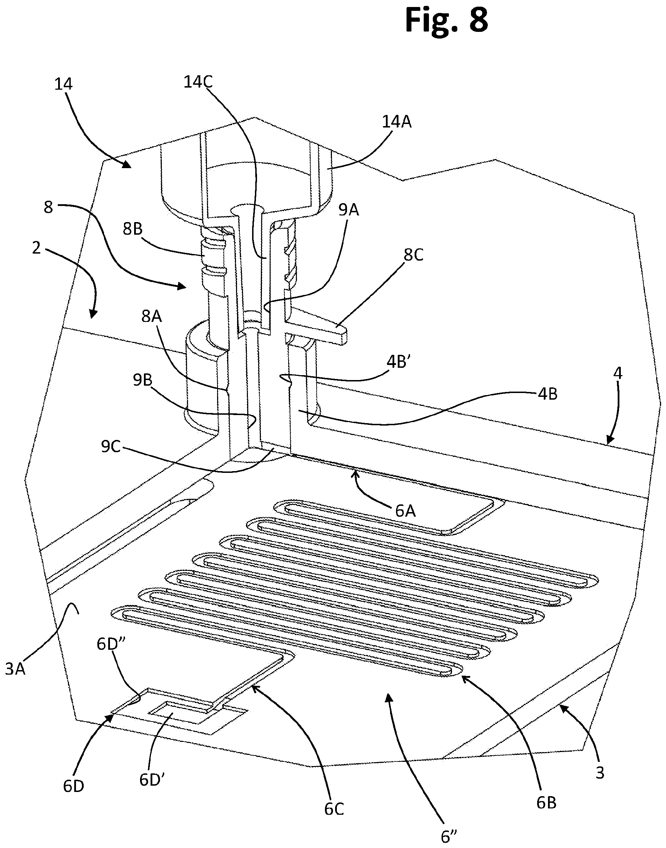

In various embodiments, the body of the deviator member 8 has a blind axial cavity 9, i.e., closed at the lower end. Referring to figures 8-10, the cavity 9 of the member 8 includes an upper part 9A, preferably but not necessarily with larger diameter, and a lower part 9B, preferably but not necessarily with smaller diameter, from which there extends - in a radial direction - a passage 9C suitable to connect the cavity 9 with each of the at least two branches 6', 6" of the fluidic circuit 6, as explained hereinafter.

Figures 3 and 8-10 show that, in preferred embodiments, the body of the deviator member 8 is substantially cylindrical-shaped, at least in a part thereof intended to be introduced in the housing formed by the hollow cylindrical parts 3B and 4B of the structures 3 and 4. Preferably, the body of the member 8 is retained inside the aforementioned housing through simple mechanical interference, for example substantially of the snap-type. For example, with reference to the case exemplified in figures 3 and 8, defined on the outer surface of the body of the member 8 is an annular relief 8A intended to be engaged in a corresponding annular recess 4B' (figure 8 only) defined in the cylindrical inner surface of part 4A.

It is to be understood that the body of the member 8 may be retained inside the aforementioned housing also in alternative ways with respect to the one represented in figure 8. For example, the body of the member 8 may have associated thereto one or more sealing elements suitable to operate on the cylindrical inner surface of part 4A, possibly engaged in one or more corresponding annular recesses defined in the wall 4A, and to maintain the position through friction, while simultaneously guaranteeing hydraulic sealing, though not limiting the rotation or the angular movement of the member 8. An example of such sealing elements consists of seals of the O-ring type.

In various embodiments, the body of the member 8 has an upper portion 8B which is shaped so as to define part of a connection, for example a connection of the luer type, for a device or system for supplying the biological sample; however, this is not an essential characteristic.

The deviator member may be displaceable in a manual or automated manner, in order to take at least two different operative configurations. With particular reference to the case illustrated in the figures, the body of the member 8 may include a part which can be operated manually, for example in the form of a radial projection 8C and/or a part which can be operated by means of an external actuation system, for example in the form of a lower axial projection 8D, preferably with a non-circular profile. As will be apparent hereinafter, the radial projection 8C may advantageously form an element for indicating the operative position of the member 8, i.e., the position of the radial passage 9C thereof (figures 8-10).

In various embodiments, the two fluidic circuit branches of the device are substantially identical to each other. Referring for example to figure 3, the two branches 6' and 6" of the circuit 6 may each include an initial portion 6A, followed by an intermediate portion 6B, in turn followed by a final portion 6C, through which the fraction of the fluid sample is conveyed to the corresponding detection member 5A or 5B. To this end, in various embodiments, the final portion 6C of each branch 6', 6" includes a transfer chamber 6D, designed to allow the fraction of the biological sample to pass through to the respective detection member 5A or 5B. To this end, in various preferred embodiments, a porous element, or in any case an element susceptible to allow the transfer of at least part of the aforementioned fraction (for example by capillarity) to the respective detection member, is arranged at each chamber 6D. With reference to figure 3, the two transfer elements are indicated with 10A, 10B. For example, as observable from figure 2, an inlet end portion 5A', 5B' of the detection member 5A, 5B extends above and at contact with the respective transfer element 10A, 10B. To this end, each element 10A, 10B may conveniently define a seat 10A', 10B' for the aforementioned inlet portion 5A', 5B' of the member 5A, 5B.

In various embodiments, the device 1 further comprises an element for accumulating the sample downstream of a respective detection member. One such accumulation element may also comprise a porous element, or in any case an element susceptible to receive at least part of the corresponding fraction of the sample (for example by capillarity) from the respective detection member. In the example shown in figures 2 and 3, the two accumulation elements are indicated with 11A and 11B. For example, as observable from figure 2, an outlet end portion 5A", 5B" of the detection member 5A, 5B extends above and at contact with the respective accumulation element 11A, 11B. Also in this case, each element 11A, 11B may conveniently define a seat for the aforementioned outlet portion 5A", 5B" of the member 5A, 5B (one such seat is visible in figure 3, indicated with 11A" for element 11A alone).

Preferably, in the surface of the structure in which there is defined the fluidic circuit - the surface 3A of the structure 3 in this case - there are defined seats 12A, 12B for the aforementioned accumulation elements 11A, 11B.

Therefore, as observable, in various embodiments the detection members extend in a bridge fashion between two porous elements, or in any case elements having a structure such to allow a liquid, particularly the biological sample, or a gas, typically air, to pass through as will be clear hereinafter. The partial superimposition of the members 5A, 5B with the respective elements 10A, 10B, on the one hand, and 11A, 11B, on the other hand, is observable in figure 2.

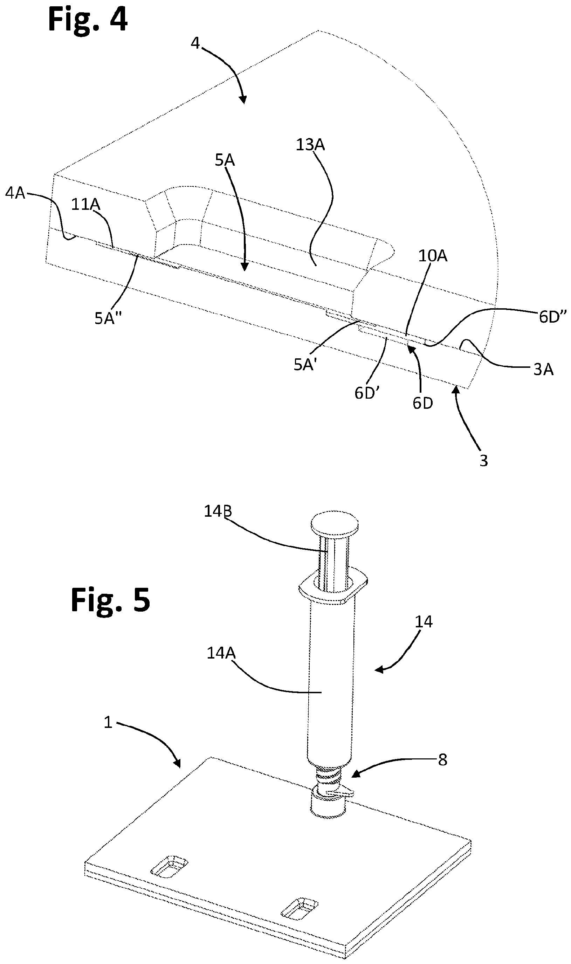

In various embodiments, the body of the detection device according to the invention is configured to allow an optical analysis of the detection members. To this end, referring for example to figures 1-3, the body 2 of the device 1 may be provided with windows 13A and 13B at the respective members 5A and 5B. In the non-limiting example, the aforementioned windows consist of through openings, particularly defined in the upper structure 4, also suitable to allow air venting. Figure 4 shows a schematic section at the window 13A, from which it can be observed that the part of the member 5A intermediate to the inlet end 5A' and the outlet 5A" end thereof is at the window itself. The same figure shows the bridging arrangement of the member 5A with respect to the elements 10A, 11A, with partial superimposition with respect to the latter. Still in figure 4, as well as in figure 3, it can be observed that, preferably, the transfer chambers 6D at the end of the circuit branches 6' and 6" have a lower part with narrowed section 6D' and an upper portion 6D" with wider section, which forms a seat for positioning the element 10A (or 10B).

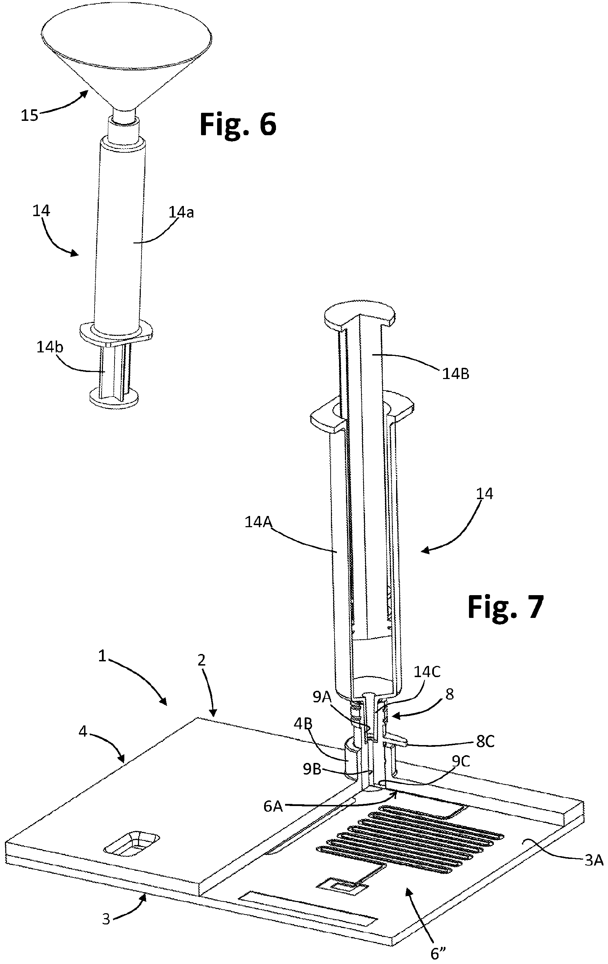

In various embodiments, the device 1 is designed to be used in combination with a further device or system configured to supply the biological sample. In various preferred embodiments, the aforementioned supply device comprises a syringe or the like, as schematised in figure 5, where a such syringe is indicated with 14 as a whole. In various embodiments, the device 1 is part of a kit also comprising the aforementioned supply device or syringe. As observable also in figures 6-7, the syringe 14 may be of the type available on the market, that is comprising a syringe cylinder 14A and a syringe plunger 14B, the cylinder having a connection 14C (figures 7-8) which alternatively serves as an inlet and as an outlet for the syringe.

In various embodiments, the aforementioned kit further comprises a collection and/or conveying device, in order to facilitate introduction of the biological sample into the introduction device herein represented by the syringe. One such collection and/or conveying device may be substantially configured in a funnel-like manner and designed to be removably coupled with the connection 14C of the syringe 14. Such case is exemplified in figure 6, where there is specifically illustrated the part of the kit including the syringe 14 with an associated funnel 15, for example for particularly advantageous use for loading a saliva sample into the syringe by the subject.

As evincible from figure 7, and even better from the detail of figure 8, the upper portion 9A of the cavity of the deviator member 8 is designed to receive the connection 14C of the syringe 14.

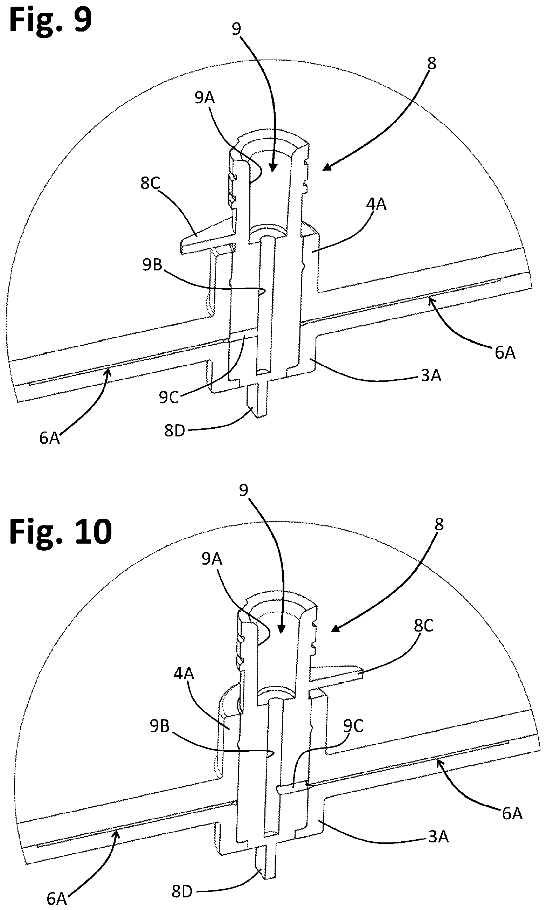

In the detail of figure 8 it is also visible how, in one of the two operative positions or configurations of the deviator member 8, the radial passage 9C of the member is axially aligned (i.e., placed in fluid communication) with the initial portion 6A of the respective circuit branch 6" (or 6'). The subsequent figures 9 and 10 both show the aforementioned positions or configurations of the member 8, which are preferably given by a 180° rotation of the member. It should be observed that, should the member 8 be designed to be actuated manually, the coupling between the member and the relative housing (formed by the parts 3B, 4B of the structures 3, 4 in this case) will preferably be configured so as to allow a user to perceive the correct angular positioning the member 8 with sufficient certainty: this can for example obtained by providing for - between the hosing and the rotatable member - an end stop or projection and recess system that allow the user to perceive a "snap" when the member is in the correct angular position.

In various embodiments, at least one from among the first branch and the second branch of the fluidic circuit comprises a coil-shaped portion, or in any case with a generally tortuous development. It is to be understood that it is possible that only one from among the first branch and the second branch comprises a coil-shaped portion. However, hereinafter in the present description reference will be made to a preferred embodiment wherein both branches 6', 6" comprise a coil-shaped portion. Actually, as mentioned, the two branches are preferably substantially identical, and this also so as to allow both fractions of the biological sample to substantially travel through a similar path through the device 1, thereby minimising the risk of introducing analysis errors due to variables in the path.

In the non-limiting examples of the figures, the aforementioned coil-shaped portions correspond to the previously indicated intermediate portions 6B of the two branches 6' and 6". Such intermediate portions 6B could also be shaped differently with respect to the exemplified one.

According to the invention, one of the first and second circuit branch 6', 6" comprises a dissociation chamber, wherein the fraction of biological sample which passes through such chamber is subjected to a dissociation treatment; such a treatment is aimed at separating complexes from among the pathogenic microorganism and the corresponding antibody possibly present in the sample fraction, so that in such fraction the pathogenic microorganism and the possibly present antibody are solely in free form. In various preferred embodiments, the dissociation chamber at least partly consists of one of the intermediate or coil-shaped portions. Hereinafter, for the sake of simplicity, reference 6B relating to the branch 6' shall therefore also be deemed to indicate the mentioned dissociation chamber, irrespective of the specific shape thereof.

The dissociation chamber may be used to carry out a thermal or chemical treatment.

In the former case, the dissociation chamber is configured to be subjected to a heating generated by a heater. The heater may be a structure physically distinct from the device 1, which is arranged in proximity of the dissociation chamber, or the heater may be integrated in the body 2 of the device 1, at the dissociation chamber.

In the latter case, the dissociation chamber contains at least one denaturing agent suitable to separate the complexes from among the pathogenic microorganism and the corresponding antibody possibly present in the biological sample; in preferred embodiments, the at least one denaturing agent is an agent capable of bringing the pH value of the biological sample to a value comprised between about 2.5 and 3, which thus allows to separate the aforementioned complexes. It is to be understood that the dissociation chamber may comprise more than a single denaturing agent.

Preferably, the denaturing agent is selected from the group consisting of HCl, Triton X-100, 3-[(3-cholamidopropyl)-dimethylammonio]-1-propanesulfonate [CHAPS]. In preferred embodiments, the denaturing agent is HCl, and such compound is capable of bringing the pH value of the biological sample to a value comprised between about 2.5 and 3.

Preferably, the at least one denaturing agent is a freeze-dried agent. The at least one denaturing agent is preferably introduced into the dissociation chamber when producing the device 1.

When using the device 1, the biological sample in which the presence of the pathogenic microorganism and/or corresponding antibody is to be detected is introduced into the device. To this end, as exemplified in figure 6, the biological sample may be initially collected in the syringe 14, for example by initially associating the funnel 15 for collecting saliva to the connection 14C thereof (figure 8). Once a sufficient amount of biological sample has been collected in the syringe 14, the funnel 15 is separated from the syringe 14.

The connection 14C of the syringe 14 is therefore coupled to the upper portion 9A of the cavity of the deviator member 8, as shown in figures 7-8.

Therefore, there is applied a pressure on the plunger 14B of the syringe 14, which is lowered so as to dispense at least part of the biological sample contained in the cylinder 14A, which thus reaches the outlet of the deviator member 8 represented by the radial passage 9C.

In this manner, should the deviator member 8 be found in the first configuration (wherein the lateral passage 9C places the lower portion 9B of the cavity 9 in communication with the initial portion 6A of the first branch 6' of the fluidic circuit 6), a first fraction of biological sample may be introduced into the fist branch 6' and advanced therealong, until it reaches a relative detection member 5A, and possibly the relative accumulation element 11A. Subsequently, the member 8 is displaced to the second configuration (in which the lateral passage 9C now places the portion 9B of the cavity 9 in communication with the initial portion 6A of the second branch 6" of the fluidic circuit 6), in order to introduce into the latter and advance a second fraction of biological sample, until it reaches the relative detection member 5B, and possibly the relative accumulation element 11B. The two alternative conditions of the deviator member 8 are shown in figures 9-10.

Obviously, should the deviator member 8 be initially found in the aforementioned second configuration, the first fraction of biological sample will be dispensed into the second branch 6" and the second fraction of biological sample will be subsequently dispensed into the first branch 6'. The two sample fractions are therefore introduced in succession in the two circuit branches.

In each branch 6', 6", each sample fraction advances until it reaches and passes through the respective coil-shaped intermediate portion 6B. As previously mentioned, one of the two circuit branches includes a dissociation chamber; in the example considered herein, such chamber corresponds to portion 6B of the branch 6'. Thus, referring to the example described herein, the first fraction of biological sample is subjected, in such chamber, to the dissociation treatment aimed at at least partly separating complexes possibly present between the pathogenic microorganism and the corresponding antibody.

In other words, after the treatment, the pathogenic microorganism and the corresponding antibody, if present, will at least partly be in free form at the outlet of the portion 6B, or in the terminal portion 6C of the branch 6'. On the contrary, the sample fraction exiting from the portion 6B of the branch 6" will not be subjected to any dissociation treatment, hence the complexes from among the pathogenic microorganism and the corresponding antibody, if present, will not be dissociated in the corresponding fraction of biological sample.

As mentioned, in various embodiments, the dissociation treatment may comprise a heating of the fraction of biological sample in the dissociation chamber by a heater, which may be a heater outside the structure of the device 1 or a heater integrated in the structure of the device, for example in the lower structure 3 of the body 2. The heating is carried out at a temperature approximately comprised between 50 °C and 95 °C, preferably between 55 °C and 65 °C, for a period of time comprised between 1 and 30 minutes, preferably between 1 and 3 minutes. Temperature increase entails the dissociation of at least part of the complexes from among pathogenic microorganism and antibody possibly present in the fraction of biological sample subjected to the thermal treatment.

As previously mentioned, the dissociation treatment may comprise a chemical treatment which is carried out on the fraction of biological sample in the dissociation chamber. As mentioned, in such embodiments, the dissociation chamber may comprise a freeze-dried denaturing agent, for example such to bring the pH value of the fraction of biological sample to a value comprised between about 2.5 and 3. In the preferred embodiment, the denaturing agent comprises HCl.

It is to be understood that it is possible that both dissociation treatments described above - thermal treatment and chemical treatment - be carried out on a fraction of biological sample. In such embodiments, the dissociation chamber is therefore provided with the denaturing agent and it is subjected to heating.

After undergoing the denaturing treatment, the first fraction of biological sample reaches the transfer chamber 6D of the relative branch 6', particularly the lower chamber portion 6D'. Even the second sample fraction will then be similarly moved toward the respective transfer chamber 6D of the relative branch 6". The advancement of each sample fraction along the relative circuit branch is obviously determined by the position of the deviator member 8 and by the actuation of the syringe 14, which generates a relative fluid pressure in the corresponding branch, with the air that can be vented through the window 13A or 13B and the porous elements represented by the elements 10A or 10B and by the detection members 5A or 5B.

As mentioned, the result of the subsequent actuations of the syringe 14, in the two switching conditions of the deviator member 8, is to bring at least part of each fraction of biological sample at the respective detection member 5A or 5B, with one of the two fractions that is subjected to a dissociation treatment. As mentioned, such detection members, preferably in form of strips, each include a binding element of the pathogenic microorganism, a binding element of the antibody and, preferably, a control element.

When at least one part of each of the two fractions of biological sample reaches at the relative member 5A or 5B, it spreads by capillarity on the aforementioned binding elements of the pathogenic microorganism and of the antibody and on the possible control element. In the case of the fraction which has travelled through the branch 6', the binding element of the pathogenic microorganism binds the possibly present pathogenic microorganism, and the binding element of the antibody binds the possibly present antibody; the control element binds anti-immunoglobulin Y (anti-IgY) or any other immune complex consisting of an antibody which is bound to the natural ligand thereof, for example albumin-anti-albumin. In the case of the fraction which has travelled through the branch 6", the binding element of the possibly present pathogenic microorganism, and the possible binding element of the antibody binds the possibly present antibody or the complexes from among the possibly present pathogenic microorganism and the antibody; the control element binds the anti-IgY or any other immune complex consisting of an antibody which is bound to the natural ligand thereof, for example albumin-anti-albumin.

By means of optical analysis, particularly using the windows 13A and 13B, it is possible to verify the conditions of the two detection members 5A and 5B, that is to detect whether the corresponding binders have bound to the aforementioned binding elements. Optical analysis also verifies the controls.

The method of use of the device 1 falls outside the scope of the invention. A possible use of the device 1 provides for the steps of:
(a) providing the biological sample,
(b) dividing the biological sample into at least one first fraction and one second fraction,
(c) carrying out a dissociation treatment on at least one of said at least one first fraction and second fraction, to separate complexes between the at least one pathogenic microorganism and the at least one corresponding antibody possibly present in such fraction,
(d) placing each of such at least one first fraction and second fraction in communication with at least one first detection member and at least one second detection member, respectively, each comprising at least one binding element of the at least one pathogenic microorganism, at least one binding element of the at least one corresponding antibody and optionally at least one control element,
(e) carrying out an optical analysis on such at least one first detection member and second detection member to detect presence of the at least one pathogenic microorganism possibly bound to the at least one binding element of the at least one pathogenic microorganism and/or of the at least one corresponding antibody possibly bound to the at least one binding element of the at least one corresponding antibody, and to detect, in the case of the fraction not subjected to the aforementioned dissociation treatment, the presence of at least one complex between the at least one pathogenic microorganism and the at least one corresponding antibody possibly bound to the at least one binding element of the at least one corresponding antibody.

As mentioned, more than one pathogenic microorganism and/or more than one corresponding antibody may be detected by means of the device according to the invention as described above.

As mentioned, in various embodiments, the first detection member and the second detection member may comprise even more than one binding element of the microorganism and more than one binding element of the corresponding antibody.

The method of use of the device 1 may comprise the further step of classifying the subject from whom the biological sample provided in step (a) comes as infected by the pathogenic microorganism if in step (e) there is detected the presence of the pathogenic microorganism at least in the fraction of biological sample on which the dissociation treatment of step (c) was carried out.

The method of use of the device 1 may comprise the further step of classifying the subject from whom the biological sample provided in step (a) comes which was classified as infected alternatively as:
- contagious, if in step (e) there is detected the presence of the pathogenic microorganism even in the fraction of biological sample on which the dissociation treatment of step (c) was not carried out, or
- not contagious or poorly contagious, if in step (e) the following conditions occur:
   (i) there is not detected the presence of the pathogenic microorganism in the fraction of biological sample on which the dissociation treatment of step (c) was not carried out, and
   (ii) there is detected the presence of the corresponding antibody in both fractions of biological sample.

The method of use of the device 1 may alternatively comprise the step of classifying the subject from whom the biological sample provided in step (a) comes as not infected by the pathogenic microorganism if in step (e) there is not detected the presence of the pathogenic microorganism at least in the fraction of biological sample on which the dissociation treatment of step (c) was carried out.

The method of use of the device 1 may comprise the step of classifying the subject from whom the biological sample provided in step (a) comes as no longer infected and not contagious if in step (e) there is detected the presence of the corresponding antibody in each of the fractions of biological sample.

As mentioned, the device according to the invention can be used to detect the presence of a virus, preferably a virus belonging to the Coronavirus family, and of a corresponding immunoglobulin selected from the group consisting of IgA, IgG and IgM. In the preferred embodiment, the device described above is used to detect the presence of the SARS-CoV-2 virus, that is the virus responsible for the COVID-19 pandemic, and the corresponding IgA.

The method of use described above, falling outside the scope of the invention, has the advantage of allowing to determine not only the presence of the infection caused by the pathogenic microorganism in the subject from whom the biological sample analysed comes, but also the level of infectivity of the subject, should the infection be present. This is particularly advantageous when the pathogenic microorganism detected is the SARS-CoV-2 virus, so it is crucial to establish whether an infected subject is contagious or not.

When a subject is infected by the SARS-CoV-2 virus, the organism triggers an antibody response in the attempt to defend itself against the viral infection. Specifically, the antibody response consists in the production of immunoglobulins. There are various types of immunoglobulins, which are produced at different times.

In particular, the first immunoglobulins produced are IgAs. They are produced at the respiratory mucous membranes, that is at an entry pathway of the SARS-CoV-2 virus of the human organism.

The IgMs appear subsequently, generally after about ten days from the infection. Lastly, the IgGs appear, after about two weeks from contact, and - unlike the IgAs and IgMs - remain in the organism even after sixty/eighty days after infection.

The activation of the immune response with ensuing production of immunoglobulins generally corresponds to a decrease in infectivity of the subject, hence, although still being infected, the subject is no longer considered susceptible of being capable of infecting other subjects. As a result, an infected subject may be considered highly contagious in the first days that elapse from the viral infection, when the virus is still present in free form and the corresponding IgAs have not yet been produced.

It is thus clear that, according to the invention, the double detection of the virus and of the IgAs in a biological sample of a subject allows not only to establish whether a subject is infected or not (by detecting the presence of the virus), but also establish whether the subject is significantly contagious (by detecting the presence of IgAs).

Should the antibody response have already been activated in the subject and in the event that, as a result, the IgAs are present in the biological sample analysed, there could be the potential risk of failing to detect the virus possibly present in the biological sample. Actually, the IgAs bind to the virus arranging themselves on the surface thereof, substantially shielding it. As a result, an optical analysis aimed at detecting the presence of the virus could be affected by the presence of IgAs and it could result in a false negative result, given that only the antibodies - but not the virus shielded by them - would be detected. Therefore, were the virus to be present but not detectable, there could be the potential risk of classifying a subject actually still infected as no longer infected.

The present inventors therefore developed the device described herein, in which the biological sample is divided into at least two fractions, one of which is subjected to a dissociation treatment aimed at separating the complexes from among the present viruses and IgAs, and the other is not subjected to any treatment. This allows also to detect possible viruses which are shielded by the presence of IgAs in the fraction of biological sample which is subjected to the dissociation treatment, therefore solving the problem described above.

Comparing the results obtained from the optical analysis carried out on the fraction of biological sample on which the dissociation treatment was carried out with the results obtained from the optical analysis caried out on the fraction of biological sample on which such treatment was not carried out, it is possible to classify the subject from whom the biological sample comes alternatively as infected and significantly contagious, infected and mildly (poorly) contagious/not contagious, no longer infected and not contagious, and not infected.

Table 1 reports the conditions required for the subject to be classified in each of these four categories, where the symbol "+" associated with the virus or with IgAs indicates the relative presence thereof in the fraction of biological sample, while the symbol "-" associated with the virus or with IgAs indicates the relative absence thereof in the fraction of biological sample.

**Table 1**

| **Fraction of biological sample subjected to the dissociation treatment** | **Fraction of biological sample not subjected to the dissociation treatment** | **Classification of the subject** |
|---|---|---|
| Virus + / IgA - | Virus + / IgA - | Infected, significantly contagious |
| Virus + / IgA + | Virus - / IgA + | Infected, mildly contagious/not contagious |
| Virus - / IgA + | Virus - / IgA + | No longer infected, not contagious |
| Virus - / IgA - | Virus - / IgA - | Not infected |

Should the virus be detected in both fractions of biological sample and IgA not be detected in any of the two fractions of biological sample, the subject is infected and significantly contagious, given that the antibody response has not yet been triggered and, as a result, it is assumed that little time has elapsed since the infection.

Should the IgA be detected in both fractions of biological sample and the virus be detected only in the fraction of biological sample subjected to the dissociation treatment, the subject is classified as infected but poorly contagious/not contagious, given that the viral infection is still present, but the antibody response has already been triggered. This means that quite some time has already elapsed since the infection, and that the infectivity of the subject is low or none. The failed detection of the virus in the fraction not subjected to dissociation is deemed a sign of the fact that the virus is shielded by IgAs.

Should the IgA be detected in both fractions of biological sample and the virus not be detected in any of the two fractions of biological sample, the subject is classified as not contagious. In particular, this is the case of a subject who has been previously infected by the virus, but who is no longer infected, given that the virus is no longer detectable. However, the antibodies are still present in the biological sample and they are evidence of the previous infection.

In cases where neither the virus nor the IgA are detected in the fractions of biological sample, the subject is not infected and has not been recently exposed to contact with the virus. Obviously, not being infected, the subject is not contagious.

As mentioned, in various embodiments, the present invention also relates to a kit comprising at least one detection device according to any one of the previously described embodiments and a device suitable to introduce the biological sample into the device, such as for example a syringe.

Preferably, the kit further comprises at least one device suitable to collect and convey the biological sample from the subject to the introduction device or syringe. In various embodiments in which the biological sample analysed is saliva, the subject may independently use the funnel for collecting the biological sample in the syringe. This reduces the risk of contaminating other subjects or the surrounding environment.

Preferably, the detection device, the introduction device or syringe and the collection device or funnel, if the latter is present, are of the disposable type, i.e., they can be used only once for a single biological sample.

An analysis apparatus can be used in combination with a device or a kit according to any one of the embodiments described above.

Generally speaking, the mentioned apparatus - which falls outside the scope of the invention - is designed to receive a detection device 1 of the previously described type, and to carry out an optical analysis at two detection members of such device, which are connected to the two branches of the fluidic circuit of the device, in which the two fractions of the same biological sample are made to pass through, one of such fractions being subjected to a dissociation treatment, as described above. The apparatus preferably includes two distinct optical detection units, each dedicated to detection on an aforementioned detection member. However, the apparatus may include a single optical unit. The optical unit may be displaceable between several detection points at which the optical analysis is carried out, or the detection device could be displaced so as to alternatively position each detection member 5A, 5B at the single optical detection unit.

The apparatus preferably has a control circuit, comprising a control logic which is configured to implement at least one classification method of the previously described type with reference to Table 1, as a function of the results of the optical analysis carried out on the two detection members.

The apparatus is designed to carry out the aforementioned dissociation treatment by supplying heat. In this case, the apparatus is provided with at least one heating device, whose operation is managed by the aforementioned control circuit.

The apparatus may be designed to receive a kit which comprises, besides the detection device 1, also a corresponding device for introducing the biological sample, for example a syringe or the like. For this case, the apparatus may advantageously be provided with a system for actuating the aforementioned introduction device or syringe. In a such case, the control circuit and logic of the apparatus are configured to manage the operation of the system for actuating the introduction device.

The apparatus may be designed for the actuation of a deviator member of the detection device, used to distribute or divide the biological sample between the two aforementioned branches of the fluidic circuit. In this case, the apparatus may be advantageously provided with a system for actuating the aforementioned deviator member. In a such case, the control circuit of the apparatus is configured to manage the operation of the system for actuating the deviator member.

The apparatus may comprise a signalling system, managed by the control logic, in order to visually and/acoustically highlight the outcome of an analysis carried out.

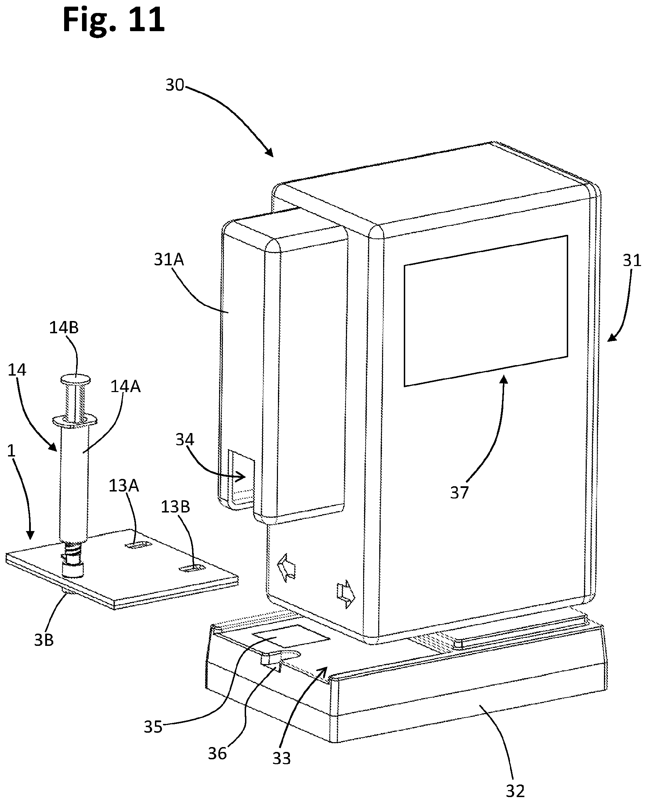

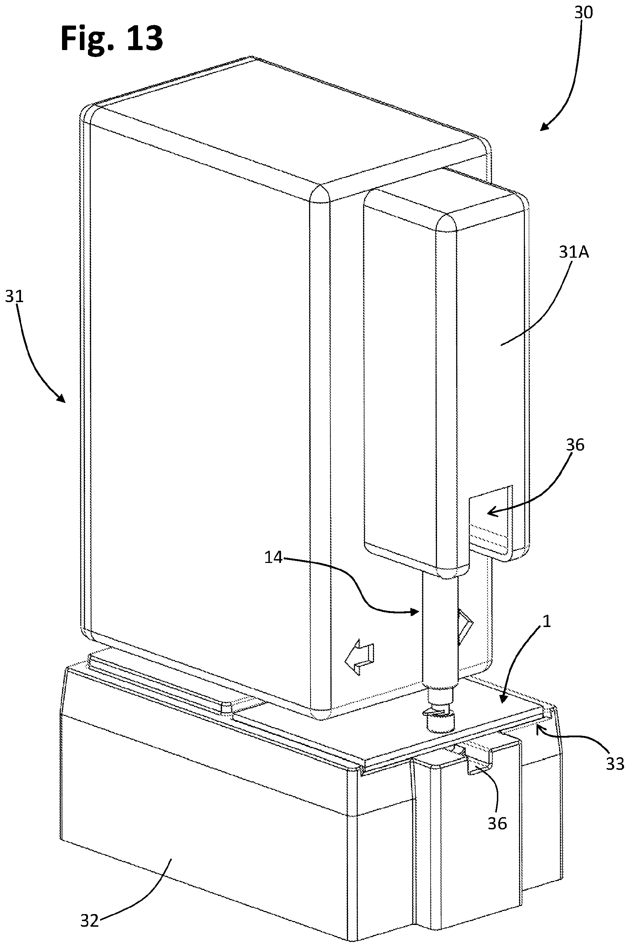

Possible layouts of an automated apparatus of the type indicated above, which falls outside the scope of the invention, are schematically exemplified in figures 11-12 and in figures 13-14, respectively, where the apparatus is represented by a stand-alone analysis machine, that is a machine integrating a plurality of the functions indicated previously. However, it should be observed that the apparatus may be formed by several machines connected together in signal communication, for example, a first machine designated to carry out optical analysis and a second machine on which the control logic is implemented and which includes a system for signalling the analysis results.

Initially with reference to figures 11-12, the machine is indicated with 30 as a whole. In the exemplified case, the machine 30 is designed to receive a kit comprising a device 1 with a syringe 14 associated therewith and for carrying out the functions of actuating the plunger 14B of the syringe 14, the functions of carrying out optical analysis on the detection members of the device 1, the heating function required to carry out the dissociation treatment, as well as the functions for processing and explaining analysis results on the device 1.

The apparatus 30 has a bearing structure or casing 31, with a base part 32 defining a seat 33 for positioning the device 1. In the front part of the structure 31 there is also defined a housing 34 suitable to receive - therein - at least the part of the syringe 14 which includes the corresponding plunger 14B.

An electric heater 35 is positioned at the seat 33, particularly at a bottom thereof. The position of the heater 35 is such that, when the device 1 is arranged in the seat 33, resting thereon is the part of the body of the device 1 in which the dissociation chamber formed by the coil-shaped portion 6B of the branch 6' of the fluidic circuit is defined (see figure 3). Preferably, in order to facilitate the transfer of heat from the heater 35 of the machine 30 to the biological sample contained in the coil-shaped portion 6B of the branch 6' of the fluidic circuit, at least the lower structure 3 of the device 1 has a reduced thickness and/or it is made with a good heat conducting material.

Furthermore, in the exemplified case, at the front part of the seat 33 there is defined a housing 36 suitable to receive the part 3B of the body of the device 1.

The apparatus preferably comprises a user interface, which in the exemplified case includes a display 37 of the touch screen type, that is suitable both for the display of information and for the allocation of commands. It goes without saying that, as an alternative, the machine 30 may include a display-only device and a plurality of electromechanical control keys. Furthermore, as mentioned, the machine 30 may be connected in signal communication with a different machine, for example a personal computer, provided with a display and with a keyboard thereof.

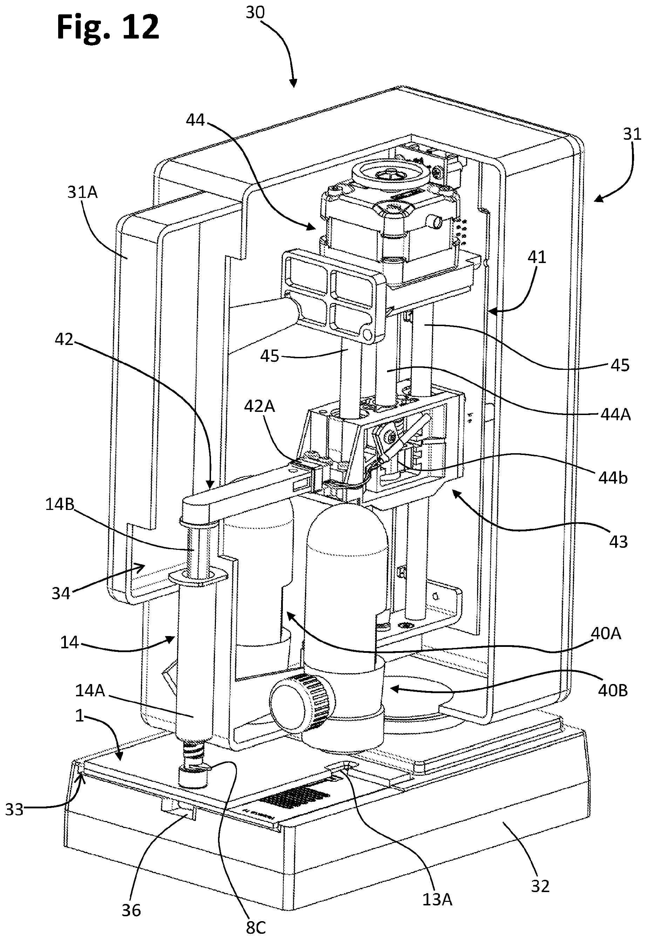

As observable in figure 12, mounted in the structure 31 are two optical detection units 40A and 40B, of a per se known design, and preferably, but not necessarily, identical to each other. The units 40A and 40B are of a type suitable to carry out the previously indicated detections at the detection members 5A and 5B of the device 1. To this end, when the device 1 is arranged in the corresponding seat 33 of the machine 30, the windows 13A and 13B of the device are axially aligned below a sensitive optical part of the units 40A and 40B, respectively. The optical units are connected in signal communication with an electronic control board 41, preferably including a microcontroller, which is part of the machine control system.

Furthermore, arranged inside the structure 31 is a system for actuating the plunger 14B of the syringe 14. In the example, movable in a hollow front part 31A of the structure 31 is an actuation member 42, suitable to controllably push the plunger of the syringe 14. As observable in figure 12, the actuation member 42 is associated with a slide 43, which is vertically movable in opposite directions and it can be actuated by means of an actuator unit 44. For example, the actuator 44 may be an electric motor with a screw shaft 44A, to which there can be associated - for example by means of a lead nut 44B - the slide 43, so that the latter may be displaced vertically in a guided manner; in the example, the slide 43 is also coupled to two vertical guide rods 45 to this end.

Obviously other possible actuation systems are possible, in order to determine the two-directional displacement of the actuation member 42 for the plunger 14B of the syringe 14.

The position of the slide 43, and therefore of the actuation member 42, can be controlled by means of suitable sensors which are part of the machine control system formed by the circuit board 41, or connected thereto. The control system, whose program or software that supervises the general operation of the machine 30, includes suitable control means, for example a programmable logic circuit or PLC or an electronic circuit with microcontroller. The control system includes the user interface represented herein by the display 37.

Still with reference to figure 12, operatively associated with the actuation member 42 there may be a sensor, for example a microswitch indicated with 42A, used for verifying the position of the slide 43. In particular, the sensor 42A has the function of detecting the mechanical contact between the member 42 and the end of the plunger 14b of the syringe 14, when the member 42 is lowered by means of the relative actuation system.

The machine 30 of figures 11-12 can be used in the case of a device 1 according to the invention, whose deviator member 8 can be actuated manually, for example using the radial projection 8C. In such cases, the member 8 does not necessarily have to be provided with the lower projection 8D. Let assume that the member 8 is in the angular position thereof corresponding to the connection of the cavity in the member with the branch 6' of the fluidic circuit of the device 1.

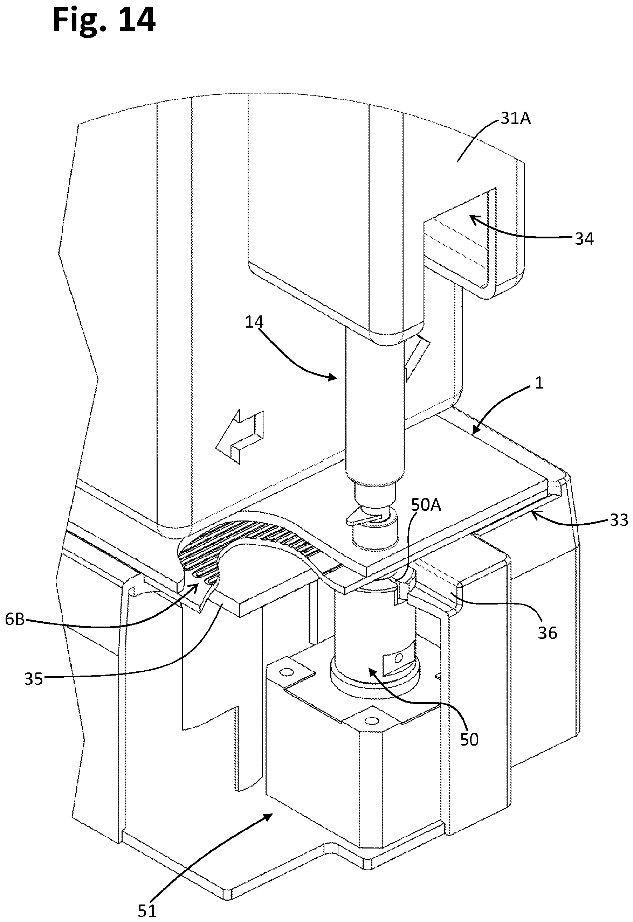

In a such case, after collecting the biological sample in the syringe 14 and after coupling the syringe to the device 1, the kit consisting of the device and syringe is arranged on the machine, as exemplified in figure 11. To this end, as mentioned, the device 1 is arranged in the corresponding seat 33, and so that the upper part of the syringe 14 enters the corresponding housing 34, below the actuation member 42 (which in such initial step will be in a raised position). As mentioned, following such positioning, the windows 13A and 13B of the device 1 will each be below the active part of the optical detection units 40A and 40B, and the coil-shaped portion 6B of the circuit branch 6', which forms the dissociation chamber, will be in a position corresponding to or slightly above the heater 35 (also see figure 14 for reference).

At this point, an analysis cycle can be started by means of the user interface 37 of the machine 30. The control logic of the machine activates the heater 35 and controls the motor unit 44, so as to cause lowering of the slide 43, and therefore of the actuation member 42. The contact between the member 42 and the top of the plunger 14B of the syringe 14 is detected by the sensor 42A: starting from such point of the movement, the control logic commands the further lowering of the member 42, by means of the slide 43, by a predetermined height, corresponding to a desired volume of the first fraction of biological sample, to be introduced into the circuit branch 6' of the device 1; when this height is reached, the lowering of the slide 43 is stopped, interrupting the operation of the relative motor unit 44.

In this manner, as it can be understood, the first fraction of the biological sample is advanced by means of the syringe along the circuit branch 6', passing through the intermediate portion 6B in which the dissociation treatment is carried out, before also passing through the relative detection member 5A, which carries out possible bonds as described above.

The control logic subsequently provides an indication, for example of the acoustic or visual type, on the user interface 37, so that the user manually switches the deviator member 8 of the device 1, i.e., he/she turns it to the angular position corresponding to the connection of the internal cavity in the member with the branch 6" of the fluidic circuit. As mentioned, the member 8 and the corresponding housing 3B-4B may be easily configured to allow a user to sense the achievement of the two switching conditions thereof. After carrying out this manual switching, the user enters a procedure resumption command through the user interface.

Therefore, the control system restarts the motor unit 44, so as to cause a further lowering of the slide 43 and therefore of the actuation member 42, i.e., determining a further lowering of the plunger 14B of the syringe 14 by a predetermined height, corresponding to a desired volume of the second fraction of biological sample, to be introduced into the circuit branch 6" of the device 1; also in this case, upon reaching such height, the lowering of the slide 43 is stopped, hence interrupting the operation of the corresponding motor unit 44.

In this manner, as can be understood, also the second fraction of the biological sample is advanced through the syringe along the circuit branch 6", until it passes through the relative detection member 5B, which operates the possible bonds as described above.

Both detection members 5A and 5B are then subjected to optical analysis by means of the detection units 40A and 40B, in order to generate signals representing the conditions of possible presence of the pathogenic microorganism and/or of the corresponding antibody described previously with reference to Table 1. These signals are processed by the control logic, which then provides for highlighting information representing the result of the processing on the user interface 37, for example according to the classification scheme exemplified in relation to Table 1, and/or to transmit the data to an external device, such as a Personal Computer.

The machine 30 may be provided with a further actuation system, which can be actuated to control the switching of the deviator member between the at least two possible operative positions in an automated manner. Such a case is exemplified in figures 13-14, in which the machine is provided, particularly at the front of the base or lower part 32 thereof, with an actuation unit 50 arranged substantially below the housing indicated with 36. In applications of this type, the member 8 is therefore equipped with the lower projection 8D.

As mentioned, the housing 36 is designed to receive the cylindrical part 3B of the body of the device 1, from which the lower projection 8D protrudes.

To this end, the projection 8D can be shaped so as to be coupled with a corresponding actuation member, preferably provided with a seat whose shape is at least partly complementary to that of said projection. An example of the aforementioned further actuation system can be seen in figure 14, where a rotary or angularly movable member, to which the projection 8D can be coupled, is indicated with 50. The actuation element 50 can be actuated by means of a corresponding actuator 51, such as an electric motor with a rotary shaft. In the illustrated example, associated with the drive shaft 51 is the member 50, whose upper part projects in the housing 36 and it has a seat or recess 50A suitable to at least partially receive the lower projection 8D of the deviator member 8, at least to an extent such to be able to impart a rotation thereto. In the example, the projection 8D is substantially in the form of a flat appendage and the seat 50A is substantially in the form of a groove, particularly with a shape suitable to allow the introduction of the device 1 and/or of the projection 8D in a direction orthogonal to the rotation or movement axis of the member 8 and/or of the actuator 50.

However, other useful forms are possible with the aim of obtaining said coupling and/or rotation of the appendage 8D, and therefore of the member 8, by means of the actuation element 50.

As can be understood, after the positioning of the kit 1, 14 on the machine 30, the control system of the latter will also independently manage the switching of the deviator member 8 of the device 1 when need arises.

Figure 15 schematically shows a detection member or strip which can be used in devices according to the invention, together with the corresponding input and output elements, in coupling and/or partial superimposing conditions.

In the example, the strip forming the detection member - in the strict sense - is indicated with 5, while the input and output elements are indicated with 10A (or 10B) and with 11A (or 11B), respectively.

The strip and the corresponding input and output elements are preferably made of one or more porous absorbent materials. Still referring to the example, at the strip 5 there are present a binding element of the at least one pathogenic microorganism, a binding element of the at least one antibody and a control element, indicated respectively with ML, AL and CE and schematised in the form of lines.

The output element 11A (or 11B) of the strip 5 consists of a second part at least partially superimposed on the central part 5, while the input element 10A (or 10B) consists of two parts 10₁ and 10₂, the part 10₁ being at least partly superimposed on the strip 5, and the part 10₂ being at least partly superimposed on the part 10₁. The parts 10₁ and 10₂ and the element 11A (or 11B) may possibly be made of a different material with respect to the strip 5.

From the description carried out above, the characteristics of the present invention are clear, same case applying to the advantages thereof. It is clear that the described devices and kits, by way of example, may be subjected to numerous variants by a person skilled in the art without departing from the scope of the invention, as defined by the claims that follow.

In embodiments alternative to those shown, the detection device may have a fluidic circuit comprising more than two branches, along which corresponding fractions of biological sample, for example three branches or four branches, are made to flow; in such cases, more than one of these paths could include a respective dissociation chamber; as a result, the apparatus or the device may include more than one heater, should the dissociation treatment be of the thermal type.

For example, with reference to the machine 30, several heaters may be provided for at the seat 33 for the device 1. Obviously, in embodiments in which the dissociation chamber of the detection device comprises at least one denaturing agent, the machine does not necessarily have to include a heater.

As previously mentioned, at least one electric heater could be integrated in the device 1, at the dissociation chamber. In a such case, at one of the outer surfaces of the body of the device, there will be accessible at least two terminals (for example in the form of electrically conductive pads), intended to be electrically connected to corresponding electrical supply terminals arranged in the seat 33, when the device 1 is arranged in such seat.

## Claims

1. A detection device for detecting presence of at least one pathogenic microorganism and/or at least one corresponding antibody in a biological sample, the detection device (1) comprising:
- a body (2),
- at least one first detection member (5A) and one second detection member (5B) on said body (2) and prearranged for binding said at least one pathogenic microorganism and/or said at least one corresponding antibody,
- at least one fluidic circuit (6) in said body (2), comprising at least one introduction portion (3B, 4B, 7) for said biological sample, wherein from the at least one introduction portion (3B, 4B, 7) there extend at least one first circuit branch (6') and a second circuit branch (6"), for receiving at least one first fraction and one second fraction of the biological sample, respectively,
wherein the first and second circuit branches (6', 6") and the first and second detection members (5A, 5B) are configured in such a way that said first fraction can reach the first detection member (5A) and said second fraction can to reach the second detection member (5B),
wherein one of the first circuit branch (6') and the second circuit branch (6") comprises a dissociation chamber (6B) for performing a dissociation of complexes between said at least one pathogenic microorganism and said at least one corresponding antibody possibly present in the biological sample.

2. The device according to Claim 1, wherein the at least one introduction portion comprises:
- at least one first and one second introduction portions, from each of which there extend the at least one first and second circuit branches (6', 6"), respectively, or else
- one and the same introduction portion from which there extend the at least one first and second circuit branches (6', 6"), or else
- one and the same introduction portion from which there extend the at least one first and second circuit branches (6', 6"), (6', 6"), wherein at said one and the same introduction portion at least one deviator device (8) is operative for addressing the first fraction of the biological sample in the first circuit branch (6') and the second fraction of the biological sample in the second circuit branch (6").

3. The device according to Claim 1 or Claim 2, wherein the body (2) is configured for enabling optical access to the first and second detection members (5A, 5B), the body (2) preferably defining a window (13A, 13B) at each detection member (5A, 5B).

4. The device according to any one of Claims 1-3, wherein at least one of the first circuit branch (6') and the second circuit branch (6") comprises at least one intermediate portion (6B) embodying at least part of said dissociation chamber (6B), said intermediate portion (6B) having preferably a generally tortuous development.

5. The device according to any one of Claims 1-4, wherein at least one of the first circuit branch (6') and the second circuit branch (6") comprises an end chamber (6D) for transferring the first fraction or the second fraction of the biological sample to the respective first detection chamber (5A) or second detection member (5A, 5B).

6. The device according to Claim 2, comprising said deviator device (8), which defines at least one from among:
a duct (9) for selective introduction of the first fraction and the second fraction of the biological sample into the first circuit branch (6') and the second circuit branch (6"), respectively;
an attachment (9A) for fluidic connection to an introduction device (14) of the biological sample;
an element (8C) for manual actuation of the deviator device (8);
an element (8C) for signalling an operative position of the deviator device (8);
an element (8D) prearranged for coupling with an external actuation system designed for driving the deviator device (8).

7. The device according to any one of Claims 1-6, wherein the body (2) comprises at least two body parts (3, 4) having mutually facing surfaces, the fluidic circuit (6) being defined at one of said surfaces.

8. The device according to any one of Claims 1-7, wherein at least one portion of the body (2) at said dissociation chamber (6B) is configured to be subjected to heating via an external heater.

9. The device according to any one of Claims 1-8, wherein the dissociation chamber (6B) contains at least one denaturing agent, preferably configured as a lyophilized compound, very preferably an agent prearranged for bringing pH of the biological sample to a value comprised between about 2,5 and 3.

10. The device according to Claim 1, comprising at least one deviator device (8) operable for addressing a first fraction of the biological sample into the first circuit branch (6') and a second fraction of the biological sample into the second circuit branch (6"), wherein the first and second circuit branches (6', 6") and the first and second detection members (5A, 5B) are configured in such way that said first fraction can reach the first detection member (5A) and said second fraction can reach the second detection member (5B) depending upon an operative position of the deviator device (8).

11. A kit comprising a detection device (1) according to any one of Claims 1-10 and a device (14) for introduction of the biological sample into the detection device (1), the device (14) for introduction of the biological sample being preferably substantially syringe-shaped, the kit preferably also comprising a device (15) for collecting and/or channelling the biological sample in the device for introduction (14).

12. A system for detecting at least one pathogenic microorganism and/or at least one corresponding antibody, comprising a detection device according to any one of Claims 1-10 and an analysis apparatus for execution of analyses of an optical type on detection members of the detection device.

## Patentansprüche

1. Nachweisvorrichtung zum Nachweisen der Anwesenheit mindestens eines pathogenen Mikroorganismus und/oder mindestens eines entsprechenden Antikörpers in einer biologischen Probe, wobei die Nachweisvorrichtung (1) umfasst:
- einen Körper (2),
- mindestens ein erstes Nachweiselement (5A) und ein zweites Nachweiselement (5B) auf dem Körper (2), die für die Bindung des mindestens einen pathogenen Mikroorganismus und/oder des mindestens einen entsprechenden Antikörpers voreingerichtet sind,
- mindestens einen Fluidikkreislauf (6) in dem Körper (2), der mindestens einen Einführungsabschnitt (3B, 4B, 7) für die biologische Probe umfasst, wobei von dem mindestens einen Einführungsabschnitt (3B, 4B, 7) mindestens ein erster Kreislaufzweig (6') und ein zweiter Kreislaufzweig (6") ausgehen, um mindestens eine erste Fraktion bzw. eine zweite Fraktion der biologischen Probe aufzunehmen,
wobei der erste und der zweite Kreislaufzweig (6', 6") und das erste und das zweite Nachweiselement (5A, 5B) so konfiguriert sind, dass die erste Fraktion das erste Nachweiselement (5A) und die zweite Fraktion das zweite Nachweiselement (5B) erreichen kann,
wobei einer von dem ersten Kreislaufzweig (6') und dem zweiten Kreislaufzweig (6") eine Dissoziationskammer (6B) zum Durchführen einer Dissoziation von Komplexen zwischen dem mindestens einen pathogenen Mikroorganismus und dem mindestens einen entsprechenden, möglicherweise in der biologischen Probe vorhandenen Antikörper umfasst.

2. Vorrichtung nach Anspruch 1, wobei der mindestens eine Einführungsabschnitt umfasst:
- mindestens einen ersten und einen zweiten Einführungsabschnitt, von denen jeweils der mindestens eine erste bzw. zweite Kreislaufzweig (6', 6") ausgehen, oder aber
- ein und derselbe Einführungsabschnitt, von dem der mindestens eine erste und der zweite Kreislaufzweig (6', 6") ausgehen, oder aber
- ein und derselbe Einführungsabschnitt, von dem der mindestens eine erste und der zweite Kreislaufzweig (6', 6"), (6', 6") ausgehen, wobei an dem ein und demselben Einführungsabschnitt mindestens eine Umlenkvorrichtung (8) wirksam ist, um die erste Fraktion der biologischen Probe in dem ersten Kreislaufzweig (6') und die zweite Fraktion der biologischen Probe in dem zweiten Kreislaufzweig (6") zu adressieren.

3. Vorrichtung nach Anspruch 1 oder Anspruch 2, wobei der Körper (2) so konfiguriert ist, dass er einen optischen Zugang zu dem ersten und zweiten Nachweiselement (5A, 5B) ermöglicht, wobei der Körper (2) vorzugsweise ein Fenster (13A, 13B) an jedem Nachweiselement (5A, 5B) definiert.

4. Vorrichtung nach einem der Ansprüche 1 bis 3, wobei mindestens einer von dem ersten Kreislaufzweig (6') und dem zweiten Kreislaufzweig (6") mindestens einen Zwischenabschnitt (6B) umfasst, der mindestens einen Teil der Dissoziationskammer (6B) verkörpert, wobei der Zwischenabschnitt (6B) vorzugsweise einen allgemein gewundenen Verlauf aufweist.

5. Vorrichtung nach einem der Ansprüche 1 bis 4, wobei mindestens einer von dem ersten Kreislaufzweig (6') und dem zweiten Kreislaufzweig (6") eine Endkammer (6D) zum Übertragen der ersten Fraktion oder der zweiten Fraktion der biologischen Probe in die jeweilige erste Nachweiskammer (5A) oder das zweite Nachweiselement (5A, 5B) umfasst.

6. Vorrichtung nach Anspruch 2, umfassend die Umlenkvorrichtung (8), die mindestens eine der folgenden Komponenten definiert:
eine Leitung (9) zum selektiven Einführen der ersten Fraktion und der zweiten Fraktion der biologischen Probe in den ersten Kreislaufzweig (6') bzw. den zweiten Kreislaufzweig (6");
einen Aufsatz (9A) zur fluidischen Verbindung mit einer Einführungsvorrichtung (14) für die biologische Probe;
ein Element (8C) zur manuellen Betätigung der Umlenkvorrichtung (8);
ein Element (8C) zur Signalisierung einer Betriebsstellung der Umlenkvorrichtung (8);
ein Element (8D), das für den Anschluss an ein externes Betätigungssystem für den Antrieb der Umlenkvorrichtung (8) voreingerichtet ist.

7. Vorrichtung nach einem der Ansprüche 1 bis 6, wobei der Körper (2) mindestens zwei Körperteile (3, 4) mit einander zugewandten Oberflächen umfasst, wobei der Fluidickreislauf (6) an einer der Oberflächen definiert ist.

8. Vorrichtung nach einem der Ansprüche 1 bis 7, wobei mindestens ein Teil des Körpers (2) an der Dissoziationskammer (6B) dazu konfiguriert ist, einer Beheizung durch eine externe Heizvorrichtung unterzogen zu werden.

9. Vorrichtung nach einem der Ansprüche 1 bis 8, wobei die Dissoziationskammer (6B) mindestens ein Denaturierungsmittel enthält, das vorzugsweise als gefriergetrocknete Verbindung konfiguriert ist, ganz besonders bevorzugt ein Mittel, das so vorbereitet ist, dass es den pH-Wert der biologischen Probe auf einen Wert zwischen etwa 2,5 und 3 bringt.

10. Vorrichtung nach Anspruch 1, umfassend mindestens eine Umlenkvorrichtung (8), die betreibbar ist, um eine erste Fraktion der biologischen Probe in den ersten Kreislaufzweig (6') und eine zweite Fraktion der biologischen Probe in den zweiten Kreislaufzweig (6") zu leiten, wobei der erste und zweite Kreislaufzweig (6', 6") und das erste und zweite Nachweiselement (5A, 5B) so konfiguriert sind, dass die erste Fraktion das erste Nachweiselement (5A) erreichen kann und die zweite Fraktion das zweite Nachweiselement (5B) erreichen kann, abhängig von einer Betriebsposition der Umlenkvorrichtung (8).

11. Kit, umfassend eine Nachweisvorrichtung (1) nach einem der Ansprüche 1-10 und eine Vorrichtung (14) zum Einbringen der biologischen Probe in die Nachweisvorrichtung (1), wobei die Vorrichtung (14) zum Einbringen der biologischen Probe vorzugsweise im Wesentlichen spritzenförmig ist, wobei das Kit vorzugsweise auch eine Vorrichtung (15) zum Sammeln und/oder Kanalisieren der biologischen Probe in der Vorrichtung zum Einführen (14) umfasst.

12. System zum Nachweis mindestens eines pathogenen Mikroorganismus und/oder mindestens eines entsprechenden Antikörpers, umfassend eine Nachweisvorrichtung nach einem der Ansprüche 1 bis 10 und ein Analysegerät zur Durchführung von Analysen optischer Art an Nachweiselementen der Nachweisvorrichtung.

## Revendications

1. Dispositif de détection pour détecter la présence d'au moins un microorganisme pathogène et/ou d'au moins un anticorps correspondant dans un échantillon biologique, le dispositif de détection (1) comprenant :
- un corps (2),
- au moins un premier élément de détection (5A) et un deuxième élément de détection (5B) sur ledit corps (2) et pré-agencés pour lier ledit au moins un microorganisme pathogène et/ou ledit au moins un anticorps correspondant,
- au moins un circuit fluidique (6) dans ledit corps (2), comprenant au moins une portion d'introduction (3B, 4B, 7) pour ledit échantillon biologique, dans lequel, à partir de l'au moins une portion d'introduction (3B, 4B, 7), s'étendent au moins une première ramification de circuit (6') et une deuxième ramification de circuit (6") pour la réception au moins d'une première fraction et d'une deuxième fraction de l'échantillon biologique, respectivement,
dans lequel les première et deuxième ramifications de circuit (6', 6") et les premier et deuxième éléments de détection (5A, 5B) sont configurés de telle sorte que ladite première fraction puisse atteindre le premier élément de détection (5A) et que ladite deuxième fraction puisse atteindre le deuxième élément de détection (5B),
dans lequel l'une parmi la première ramification de circuit (6') et la deuxième ramification de circuit (6") comprend une chambre de dissociation (6B) pour la mise en oeuvre d'une dissociation de complexes entre ledit au moins un microorganisme pathogène et ledit au moins un anticorps correspondant éventuellement présent dans l'échantillon biologique.

2. Dispositif selon la revendication 1, dans lequel l'au moins une portion d'introduction comprend :
- au moins une première et une deuxième portions d'introduction, à partir de chacune desquelles s'étendent les au moins une première et deuxième ramifications de circuit (6', 6"), respectivement, ou sinon
- une seule et même portion d'introduction à partir de laquelle s'étendent les au moins une première et deuxième ramifications de circuit (6', 6"), ou sinon
- une seule et même portion d'introduction à partir de laquelle s'étendent les au moins une première et deuxième ramifications de circuit (6', 6"), (6', 6"), dans laquelle, au niveau de dite seule et même portion d'introduction, au moins un dispositif déviateur (8) est fonctionnel pour adresser la première fraction de l'échantillon biologique dans la première ramification de circuit (6') et la deuxième fraction de l'échantillon biologique dans la deuxième ramification de circuit (6").

3. Dispositif selon la revendication 1 ou la revendication 2, dans lequel le corps (2) est configuré pour permettre un accès optique aux premier et deuxième éléments de détection (5A, 5B), le corps (2) définissant de préférence une fenêtre (13A, 13B) au niveau de chaque élément de détection (5A, 5B).

4. Dispositif selon l'une quelconque des revendications 1 à 3, dans lequel au moins l'une parmi la première ramification de circuit (6') et la deuxième ramification de circuit (6") comprend au moins une portion intermédiaire (6B) incorporant au moins une partie de ladite chambre de dissociation (6B), ladite portion intermédiaire (6B) ayant de préférence un développement globalement tortueux.

5. Dispositif selon l'une quelconque des revendications 1 à 4, dans lequel au moins l'une parmi la première ramification de circuit (6') et la deuxième ramification de circuit (6") comprend une chambre d'extrémité (6D) pour transférer la première fraction ou la deuxième fraction de l'échantillon biologique vers la première chambre de détection (5A) ou le deuxième élément de détection (5A, 5B) respectif.

6. Dispositif selon la revendication 2, comprenant ledit dispositif déviateur (8), qui définit au moins l'un parmi :
un conduit (9) pour l'introduction sélective de la première fraction et de la deuxième fraction de l'échantillon biologique dans la première ramification de circuit (6') et la deuxième ramification de circuit (6") respectivement ;
un accessoire (9A) pour une connexion fluidique à un dispositif d'introduction (14) de l'échantillon biologique ;
un élément (8C) pour l'actionnement manuel du dispositif déviateur (8) ;
un élément (8C) pour signaler une position opérationnelle du dispositif déviateur (8) ;
un élément (8D) pré-agencé pour un couplage avec un système d'actionnement externe conçu pour entraîner le dispositif déviateur (8).

7. Dispositif selon l'une quelconque des revendications 1 à 6, dans lequel le corps (2) comprend au moins deux parties de corps (3, 4) ayant des surfaces se faisant mutuellement face, le circuit fluidique (6) étant défini au niveau de l'une desdites surfaces.

8. Dispositif selon l'une quelconque des revendications 1 à 7, dans lequel au moins une portion du corps (2) au niveau de ladite chambre de dissociation (6B) est configurée pour être soumise à un chauffage via un élément chauffant externe.

9. Dispositif selon l'une quelconque des revendications 1 à 8, dans lequel la chambre de dissociation (6B) contient au moins un agent dénaturant, de préférence configuré sous la forme d'un composé lyophilisé, mieux encore un agent pré-agencé pour porter le pH de l'échantillon biologique à une valeur comprise entre environ 2,5 et 3.

10. Dispositif selon la revendication 1, comprenant au moins un dispositif déviateur (8) fonctionnel pour adresser une première fraction de l'échantillon biologique dans la première ramification de circuit (6') et une deuxième fraction de l'échantillon biologique dans la deuxième ramification de circuit (6"), dans lequel les première et deuxième ramifications de circuit (6', 6") et les premier et deuxième éléments de détection (5A, 5B) sont configurés de telle sorte que ladite première fraction puisse atteindre le premier élément de détection (5A) et que ladite deuxième fraction puisse atteindre le deuxième élément de détection (5B) en fonction de la position opérationnelle du dispositif déviateur (8).

11. Kit comprenant un dispositif de détection (1) selon l'une quelconque des revendications 1 à 10 et un dispositif (14) pour l'introduction de l'échantillon biologique dans le dispositif de détection (1), le dispositif (14) pour l'introduction de l'échantillon biologique étant de préférence sensiblement en forme de seringue, le kit comprenant aussi un dispositif (15) pour collecter et/ou canaliser l'échantillon biologique dans le dispositif aux fins d'une introduction (14).

12. Système pour détecter au moins un microorganisme pathogène et/ou au moins un anticorps correspondant, comprenant un dispositif de détection selon l'une quelconque des revendications 1 à 10 et un appareil d'analyse pour l'exécution d'analyses de type optique sur des éléments de détection du dispositif de détection.
